# EUROPÄISCHE PATENTSCHRIFT

(11) **EP 1 128 812 B1**
(45) Veröffentlichungstag und Bekanntmachung des Hinweises auf die Patenterteilung: **25.02.2004**
(21) Anmeldenummer: 99955981.8
(22) Anmeldetag: 12.11.1999
(51) Int. Cl.: A61K 9/107, A61K 31/197, A61K 41/00, A61P 35/00, A61P 17/06

(54) **5-AMINOLÄVULINSÄURE-NANOEMULSION**
NANO-EMULSION OF 5-AMINOLEVULINIC ACID
EMULSION NANOMETRIQUE D'ACIDE 5-AMINOLEVULINIQUE

(30) Priorität: 12.11.1998 DE 19852245
(43) Veröffentlichungstag der Anmeldung: 05.09.2001
(73) Patentinhaber: Asat AG Applied Science & Technology, 6302 Zug (CH)
(72) Erfinder: Schmid, Hans W., 6303 Zug (CH); Burmeister, Gerd, 4455 Zunzgen (CH)
(74) Vertreter: Weiss, Wolfgang, Dipl.-Chem. Dr.
(86) Internationale Anmeldenummer: PCT/EP1999/008711
(87) Internationale Veröffentlichungsnummer: WO 2000/028971

(56) Entgegenhaltungen:
- EP-A- 0 274 431
- EP-A- 0 704 209
- US-A- 5 152 923
- HÜRLIMANN AF, ET AL.: "Photodynamic therapy of superficial basal cell carcinomas using topical 5-aminolevulinic acid in a nanocolloid lotion" DERMATOLOGY, Bd. 197, Nr. 3, 1998, Seiten 248-254, XP000884929 ISSN 1018-8665

## Beschreibung

Die vorliegende Erfindung betrifft Nanoemulsionen, welche 5-Aminolävulinsäure, Derivate, Vorstufen oder Metaboliten davon enthalten.

Die photodynamische Therapie ist eine neue und vielversprechende Methode zur Behandlung von verschiedenen prämalignen und malignen Erkrankungen, die mit der Zellproliferation in Zusammenhang stehen. Das Prinzip der photodynamischen Therapie beruht darauf, einen sog. Photosensibilisator in das Tumorgewebe einzubringen und diesen durch Bestrahlung mit Licht geeigneter Wellenlänge in einem cytotoxisch aktiven Wirkstoff umzuwandeln, welcher letztlich die Zerstörung der Zellen bewirkt. Die Selektivität dieser Methode beruht in einer stärkeren Anreicherung des Sensibilisators in schnell proliferierenden Tumorzellen im Vergleich zum Normalgewebe. Durch eine lokal begrenzte Lichtbestrahlung kann der in den Tumorzellen enthaltene Sensibilisator gezielt aktiviert werden, wodurch die Krebszellen unter weitgehender Schonung des gesunden Gewebes zerstört werden.

Bisher wurde als Photosensibilisator zumeist ein intravenös verabreichbares Gemisch von Haematoporphyrinderivaten verwendet. Trotz ermutigender klinischer Erfolge bei untschiedlichen Krebsarten weisen diese Haematoporphyrinderivate jedoch verschiedene Nachteile auf. Erstens treten infolge der geringen Tumorselektivität und der nur langsamen Elimination aus dem Körper relativ hohe Wirkstoffkonzentrationen im Normalgewebe auf. Demzufolge finden bei der Bestrahlung unerwünschte photochemische Reaktionen im gesunden Gewebe statt. Zweitens resultiert diese Behandlung in einer allgemeinen Lichtempfindlichkeit, so daß sich der Patient während etwa vier Wochen nicht dem Tageslicht aussetzen darf.

Eine Verringerung der hohen Wirkstoffkonzentration im Normalgewebe und somit der unerwünschten Nebenwirkungen kann in bestimmten Fällen, insbesondere bei dermatologischen und gynäkologischen Anwendungen durch die Entwicklung topisch applizierbarer Wirkstoffformulierungen anstelle der bekannten systemischen Formulierungen erreicht werden. Zur Verringerung der Lichtempfindlichkeit wird weiterhin versucht, Precursoren von Photosensibilisatoren einzusetzen, die photochemisch inaktiv sind und erst innerhalb der Zielzelle in einen Photosensibilisator umgewandelt werden.

5-Aminolävulinsäure ist eine körpereigene Substanz, die aus Glycin und Succinyl-CoA synthetisiert wird. Im Rahmen der Haem-Biosynthese entsteht aus 5-Aminolävulinsäure (5-ALA) in mehreren schnell verlaufenden Reaktionsschritten das hochgradig photoaktive Protoporphyrin IX, das anschließend in einer langsamen Reaktion zur Haem umgewandelt wird. Ein natürlicher Regelmechanismus hemmt bei zu hoher Haemkonzentration sowohl die körpereigene Synthese von 5-Aminolävulinsäure als auch den Abbau von Protoporphyrin IX.

Durch die exogene Verabreichung von synthetisch hergestellter 5-Aminolävulinsäure wird dieser Regelmechanismus umgangen, was zu einer erhöhten Produktion von Protoporphyrin IX führt. Da dessen Abbau durch den natürlichen Kontrollmechanismus weiterhin gehemmt ist, reichert sich das Protoporphyrin IX in den Zellen an. Protoporphyrin IX kann bei Bestrahlung mit Licht eine photochemische Oxidationsreaktion eingehen und wirkt somit als Photosensibilisator. Bei Absorption eines Lichtquants durch das Sensibilisatormolekül wird dieses zunächst in einen elektronisch angeregten Zustand (Singulettzustand) versetzt, welcher relativ kurzlebig ist und seine Überschußenergie entweder innerhalb einiger Nanosekunden durch Emission eines Fluoreszenzphotons wieder abgibt oder in einen relativ langlebigen Triplettzustand übergeht. Aus diesem Triplettzustand kann Energie auf in der Zelle vorhandene Sauerstoffmoleküle übertragen werden.

Der dabei entstehende Singulett-Sauerstoff wirkt cytotoxisch, insbesondere auf proliferierende Zellen, da er mit Zellkomponenten, z.B. der Zellmembran und Mitochondrien, reagiert oder die Bildung von zellschädigenden Radikalen auslöst. Weiterhin führt die Bestrahlung des Photosensibilisators zu einer charakteristischen Fluoreszenzstrahlung, welche für Nachweisreaktionen, beispielsweise zum Nachweis proliferierender Zellen verwendet werden kann.

Aus dem Stand der Technik sind eine Reihe von Untersuchungen unter Verwendung topisch applizierbarer 5-Aminolävulinsäure-Zusammensetzungen bekannt. Diesen Untersuchungen ist gemeinsam, daß die eingesetzte 5-Aminolävulinsäure in Form einer Öl-in-Wasser-Emulsion vorliegt, Unterschiede bestehen jedoch hinsichtlich weiterer Parameter, wie Penetrationsdauer, Behandlungsdauer, Art des verwendeten Lichts und aufgebrachte Lichtdosis.

B. Thiele et al. (H+G, Band 69, Heft 3, Seiten 161-164 (1994)) beschreiben Untersuchungen unter Verwendung von 20% δ-Aminolävulinsäure in Form einer Öl-in-Wasser-Emulsion bei einer Penetrationsdauer von 5 bis 6 h und anschließender Bestrahlung mittels eines Argonionengepumpten Farbstofflasers (Emissionsmaximum 630 nm) mit einer kumulativen Gesamtdosis von 50 bis 100 J/cm².

Wolf et al. (Journal of the American Academy of Dermatology Vol. 28, Seiten 17 bis 21, 1993) beschreiben Untersuchungen unter Verwendung von 20% 5-Aminolävulinsäure in Form einer Öl-in-Wasser-Emulsion mit einer Penetrationsdauer von 4, 6 oder 8 h und Bestrahlung mittels unfiltriertem Licht oder Rotlicht mit einer Lichtdosis von 30 J/cm² bis zu 100 J/cm².

Obwohl die aus dem Stand der Technik bekannten Untersuchungen das vielversprechende Potential der photodynamischen Therapie unter Verwendung von 5-Aminolävulinsäure klar aufzeigen, sind bisher bekannte Öl-in-Wasser-Emulsionen mit einer Reihe von Nachteilen behaftet.

So wurde von M. Novo Rodriguez et al. (SPIE, Vol. 2371, Seiten 204-209) gezeigt, daß Aminolävulinsäure in den für eine klinische Anwendung erforderlichen hohen Konzentrationen im neutralen bis basischen pH-Bereich in wässrigen Lösungen instabil ist. Lediglich bei einem pH-Wert von 5,01 werden im untersuchten Zeitraum von 25 h zufriedenstellende Ergebnisse erhalten, und als optimale Bedingungen für wässrige 5-Aminolävulinsäurelösungen wird eine Konzentration von 3% und ein pH-Wert von 5 angegeben. Für eine klinische Anwendung wird es jedoch im allgemeinen erforderlich sein, auch Zusammensetzungen in einem höheren Konzentrationsbereich zur Verfügung zu stellen, darüber hinaus ist für eine kommerzielle Anwendung eine Stabilität der 5-ALA-Lösungen in einer Größenordnung von Wochen oder Monaten erforderlich.

V. von Arx et al. (J. Pharm. Pharmacol. 49: 652-656, 1997) beschreiben Untersuchungen zur topischen Applikation von 5-Aminolävulinsäure in verschiedenen Gelen. Als beste Formulierung zur Aufrechterhaltung der Stabilität von 5-Aminolävulinsäure wird eine Kombination mit Novion AA-1, einer Polyacrylsäure, bei einem pH < 6 angegeben.

Hürlimann et al. (Dermatology, Band 197, Nr. 3 1998, Seiten 248-254) offenbaren Nanokolloid-Lotionen, die 5-Aminolävulinsäure enthalten, sowie ihre Verwendung bei der photodynamischen Therapie. Jene Nanokolloide aus dem Stand der Technik wurden jedoch unter Verwendung von Eilecithin als Emulgator hergestellt.

Ein weiterer Nachteil der bekannten Öl-in-Wasser-Emulsionen besteht darin, daß die Eindringtiefe des Photosensibilisators in das geschädigte Gewebe nicht optimal ist. Dadurch ist in vielen Fällen das erkrankte Gewebe nur in seinen oberflächigen Schichten der photodynamischen Therapie zugänglich, obwohl die Eindringtiefe des zur Aktivierung des Photosensibilisators verwendeten Lichts auch eine Behandlung tiefer liegender Schichten ermöglichen würde.

Aufgabe der vorliegenden Erfindung war es somit, 5-Aminolävulinsäure enthaltende Zusammensetzungen bereitzustellen, bei denen die aus dem Stand der Technik bekannten Nachteile zumindest teilweise beseitigt sind und die insbesondere eine ausreichende Stabilität besitzen und eine verbesserte Eindringtiefe in Gewebe zeigen.

Gelöst wird diese Aufgabe durch eine Zusammensetzung, welche dadurch gekennzeichnet ist, daß sie eine Nanoemulsion umfassend eine Wirksubstanz ausgewählt aus 5-Aminolävulinsäure, einem Salz, einem Komplex oder einer Additionsverbindung davon, oder/und zu Protoporphyrin IX umsetzbaren Vorstufen oder/und einem Metaboliten davon und einen Träger in einer wässrigen Phase enthält, wober der Träger aus einem oder mehreren Lipiden und aus einem oder mehreren Emulgatoren umfassend Sojalecithin gebildet ist.

Überraschenderweise wurde festgestellt, daß die Stabilität von 5-Aminolävulinsäure bei Formulierung in eine Nanoemulsion erheblich gesteigert werden kann. Die Ursachen hierfür sind nicht bekannt, es scheint jedoch, daß ein vom Nanosomen geschaffenes Microenvironment sich besonders günstig auf die Stabilität der 5-Aminolävulinsäure auswirkt.

Weiterhin hat sich überraschend gezeigt, daß mit den erfindungsgemäßen Nanoemulsionen sehr hohe Gewebeeindringtiefen erreicht werden können, wordurch auch tiefer liegende Erkrankungen bzw. Erkrankungen mit höheren Schichtdicken der Behandlung zugänglich werden. Die größeren Eindringtiefen waren insbesondere deswegen überraschend, weil bisher davon ausgegangen worden war, daß 5-Aminolävulinsäure aufgrund ihrer geringen Größe ohnehin leicht durch eine geschädigte Epidermis, die beispielsweise über Entzündungen, Präkanzerosen und Tumoren vorliegt, eindringen kann.

Ein dritter überraschender Vorteil liegt darin, daß die erfindungsgemäß in Nanosomen verpackte 5-Aminolävulinsäure offenbar sehr gut von den Zellen aufgenommen wird. Dadurch wird einerseits ein verbessertes Targeting erreicht, und andererseits kann die Penetrationsdauer, d.h. der Zeitraum zwischen dem Aufbringen der Zusammensetzung und der Lichtbestrahlung des erkrankten Gewebes reduziert werden, was für den Patienten eine spürbare Erleichterung darstellt.

Erfindungsgemäß umfaßt die Nanoemulsion eine Wirksubstanz ausgewählt aus 5-Aminolävulinsäure, einem Derivat, einer Vorstufe oder/und einem Metaboliten davon. Unter "Derivat" sind insbesondere Salze, Komplexe und Additionsverbindungen zu verstehen. Unter "Vorstufe" und "Metabolit" sind dabei solche Substranzen zu verstehen, die in einer Zelle zu Protoporphyrin IX umgesetzt werden. Besonders bevorzugt ist die Wirksubstanz 5-Aminolävulinsäure oder ein Derivat davon. Der Träger kann ein beliebiger aus einem oder mehreren Lipiden und aus einem oder mehreren Emulgatoren, umfassend Sojalecithin, gebildeter Träger sein, solange er zur Ausbildung der Nanoemulsion in einer wässrigen Phase fähig ist. Vorzugsweise umfaßt der Träger eine Ölphase, d.h. ein nicht mit Wasser mischbares Material, z.B. Lipide, sowie einen Emulgator. Zweckmäßigerweise werden physiologisch unbedenkliche Trägersubstanzen eingesetzt.

Die Größe der emulgierten Teilchen in der Nanoemulsion (Nanosomen) beträgt im Mittel ≤ 200 nm, z.B. 10 bis 200 nm. Die jeweils optimale Teilchengröße hängt von weiteren Parametern, wie beispielweise der Viskosität der Zusammensetzung ab. Beispielsweise wurden mit einem Gel mit einer Viskosität von 5 mPas bei einem mittleren Teilchendurchmesser von etwa 110 nm gute Ergebnisse erhalten und ebenfalls für eine Lotion mit einer Viskosität von 1,6 mPas bei einem mittleren Teilchendurchmesser von etwa 20 nm.

Geeignete Trägersysteme, welche über einen langen Zeitraum stabil sind, keine hohen Konzentrationen von Tensiden und Cotensiden enthalten und frei von toxischen Emulgatorkomplexen sind, werden z.B. im US-Patent 5,152,923 offenbart. Diese Nanoemulsionen umfassen als Emulgator ein Glycerophosphatid wie beispielweise ein Lecithin oder ein Cephalin und als Ölphase physiologisch verträgliche Lipide, z.B. Triglyceride, wie etwa pflanzliche oder tierische Öle, z.B. Erdnußöl, Sojabohnenöl etc. Das Gewichtsverhältnis von Emulgator/Öl beträgt von 0,05 bis 0,4:1.

Emulgatoren, welche in der Praxis bereits in 5-Aminolävulinsäure-Nanoemulsionen erfolgreich eingesetzt worden sind, sind beispielsweise Eilecithin, Sojalecithin und Phosphatidylcholin. Ein bewährtes Lipid ist beispielsweise Miglyol 812.

Der Anteil der Wirksubstanz, z.B. 5-Aminolävulinsäure in der Zusammensetzung hängt im wesentlichen von dem vorgesehenen Anwendungszweck ab. Im allgemeinen sind etwa 1 bis 25 Gew.-%, bezogen auf das Gesamtgewicht der Zusammensetzung, vorhanden. Höhere bzw. niedrigere Dosierungen sind jedoch machbar. Für Anwendungen im Zusammenhang mit photodynamischer Therapie hat sich ein Anteil von 5 bis 15 Gew.-%, insbesondere von etwa 10 Gew.-% als geeignet erwiesen.

Die Zusammensetzung kann weiterhin Hilfs- oder/und Zusatzstoffe umfassen und insbesondere solche Stoffe, welche in der Kosmetik oder Pharmazie üblich sind. Beispiele für derartige Substanzen sind etwa Puffer, Stabilisatoren, zusätzliche Emulgatoren, Verdickungsmittel, etc.

In einer besonders bevorzugten Ausführungsform ist die erfindungsgemäße Zusammensetzung ein Gel, welches, bezogen auf das Gesamtgewicht der Zusammensetzung 1 bis 25 Gew.-%, bevorzugt 5 bis 15 Gew.-% Wirksubstanz, 40 bis 60 Gew.-%, bevorzugt 45 bis 55 Gew.-% Träger, 0 bis 10 Gew.-%, bevorzugt 1 bis 5 Gew.-% Hilfsstoffe und den Rest Wasser umfaßt.

Gemäß einer weiteren besonders bevorzugten Ausführungsform ist die erfindungsgemäße Zusammensetzung eine Lotion, welche, bezogen auf das Gesamtgewicht der Zusammensetzung, 1 bis 25 Gew.-%, bevorzugt 5 bis 15 Gew.-% Wirksubstanz, 10 bis 30 Gew.-%, bevorzugt 15 bis 25 Gew.-% Träger, 10 bis 30 Gew.-%, bevorzugt 15 bis 25 Gew.-% Hilfsstoffe und den Rest Wasser umfaßt.

Wie eingangs erwähnt, zeigt die erfindungsgemäße 5-Aminolävulinsäure-Zusammensetzung eine überraschend hohe Lagerstabilität, wobei der Anteil an Wirksubstanz in einer Zusammensetzung, die einen pH-Wert zwischen 1,5 und 3 aufweist, nach einjähriger Lagerung bei Raumtemperatur vorzugsweise um nicht mehr als 5% und besonders bevorzugt nicht mehr als 4% verringert ist. Nach einjähriger Lagerung bei 5°C ist der Anteil an Wirksubstanz vorzugsweise um nicht mehr als 3% und besonders bevorzugt um nicht mehr als 2,5% verringert.

Ein weiterer Gegenstand der vorliegenden Erfindung ist eine erfindungsgemäße Zusammensetzung in Form eines pharmazeutischen Präparats. In diesem Fall ist die Zusammensetzung frei von Bestandteilen, die pharmazeutisch nicht akzeptabel sind und bevorzugt frei von Bestandteilen, welche beispielweise Irritationen hervorrufen. Das pharmazeutische Präparat kann neben den bereits genannten Trägersubstanzen weitere Hilfs- oder/und Zusatzstoffe enthalten, die akzeptabel und bevorzugt gut verträglich sind.

Das pharmazeutische Präparat kann in einer Form vorliegen, die für eine systemische Verabreichung geeignet ist, wie beispielsweise eine injizierbare Flüssigkeit. Für dermatologische und gynäkologische Anwendungen liegt das Präparat jedoch bevorzugt in einer Form vor, die für eine topische Applikation geeignet ist. Das Präparat weist für die jeweils gewünschte Applikationsform günstige Eigenschaften, z.B. Viskosität und Rheologie auf, um zu gewährleisten, daß nach der Applikation ein ausreichendes Eindringen der mit 5-Aminolävulinsäure beladenen Nanosomen in das Zielgewebe erfolgt. Diese Viskositäts- und Rheologieeigenschaften können durch Zugabe von Verdickungsmitteln wie beispielsweise Polyethylenglykolstearylethern, Polyethylenglykolstearaten oder/und Polysacchariden wie etwa Polysaccharid B-1459, eingestellt werden.

Ein weiterer Gegenstand der vorliegenden Erfindung ist ein Verfahren zur Herstellung der erfindungsgemäßen Zusammensetzung bzw. des pharmazeutischen Präparats. Dabei werden die Bestandteile des Trägermaterials in einer wässrigen Phase vorgelegt und das Gemisch durch intensive Homogenisierung in eine Nanoemulsion überführt. Hierzu können beispielsweise kommerziell erhältliche Hochdruck-Homogenisatoren eingesetzt werden. Die 5-Aminolävulinsäure und gegebenenfalls vorhandene Zusatzstoffe können vor oder/und nach der Homogenisierung zugegeben werden. Nach Herstellung der Nanoemulsion können weitere Hilfs- und Zusatzstoffe, deren Gegenwart bei der Homogenisierung nicht erwünscht war, zugegeben werden.

Bevorzugt wird das Verfahren unter Luftausschluß durchgeführt, beispielsweise mittels Anlegen eines Vakuums oder/und einer Schutzgasatmosphäre. Außerdem ist es bevorzugt, unter Lichtausschluß zu arbeiten. Das Verfahren wird bei einer Temperatur durchgeführt, bei der die Ausbildung der gewünschten Nanoemulsion stattfinden kann und eine ausreichende Stabilität der Bestandteile, insbesondere der Wirksubstanz gegeben ist. Im allgemeinen hat sich ein Temperaturbereich von etwa 5 bis 45°C als geeignet herausgestellt. Die Verarbeitung von Hilfs- oder/und Zusatzstoffen, die beispielsweise zunächst in einem separaten Ansatz vermischt und gegebenenfalls homogenisiert werden, und erst danach zu der Zusammensetzung zugegeben werden, kann jedoch auch bei höheren Temperaturen, beispielsweise bis etwa 80°C erfolgen. Für eine pharmazeutische Anwendung wird für eine Sterilität des entstehenden Produkts gesorgt, z.B. durch Einsatz steriler Ausgangsmaterialien und Einhaltung steriler Verfahrensbedingungen oder/und durch einen Sterilisationsschritt nach der Herstellung.

Ein wesentliches Verwendungsgebiet für die erfindungsgemäßen Zusammensetzungen liegt auf dem Gebiet der photodynamischen Therapie; wobei die Nanoemulsion besonders bevorzugt topisch appliziert wird. Der Einsatz der erfindungsgemäßen Nanoemulsion ist möglich bei sämtlichen Erkrankungen, deren Bekämpfung eine Proliferationshemmung oder eine Abtötung von Zellen oder Gewebe mittel Photoaktivierung eines aus 5-Aminolävulinsäure gebildeten Sensibilisators umfaßt. Hierzu zählen insbesondere Erkrankungen, die mit einer gesteigerten Zellproliferation assoziiert sind, da in diesem Fall eine besonders hohe Anreicherung des Photosensibilisators durch den gesteigerten Zellmetabolismus in erkrankten Zellen stattfindet.

Die erfindungsgemäßen Zusammensetzungen sind demnach geeignet zur Behandlung von Tumorerkrankungen, wie beispielsweise Basalzellkarzinome, Plattenepithelkarzinome, Morbus Bowen, aktinische Keratose, Condylomata acuminata (CIN), epitheliale Neoplasie der Vulva (VIN), knotenartige und subkutane Krebserkrankungen. Ein Beispiel für eine nichttumorartige Erkrankung ist etwa Psoriasis.

Die Behandlung erfolgt z.B. durch topische Applikation einer die Wirksubstanz, z.B. 5-Aminolävulinsäure enthaltenden Nanoemulsion und anschließende Inkubation, um das Eindringen einer ausreichenden Menge der 5-Aminolävulinsäure in das zu behandelnde Gewebe zu gestatten. Während der Inkubation wird eine Lichteinstrahlung auf die behandelte Stelle bevorzugt z.B. durch Abdecken vermieden, um eine unerwünschte vorzeitige Aktivierung zu verhindern. Nach Ablauf des Inkubationszeitraumes, der im Allgemeinen etwa 1 bis 8 h, und üblicherweise etwa 4 h beträgt, wird das Gewebe mit einer Lichtquelle in einer ausreichenden Strahlungsdosis bestrahlt. Geeignete Lichtquellen umfassen Lampen, welche weißes Licht abgeben, sowie monochromatische Lichtquellen, wie etwa einen Laser, insbesondere Argon-Farbstofflaser mit einer Emission bei etwa 630 nm. Die Strahlungsdosen liegen üblicherweise in einem Bereich von etwa 20 J/cm² bis mehrere 100 J/cm² pro Anwendung.

Ein weiteres Verwendungsgebiet für die erfindungsgemäßen Nanoemulsionen betrifft den Nachweis des Vorhandenseins von proliferierenden Zellen in einer Probe, z.B. einer Gewebeprobe. Der Nachweis beruht auf einer selektiven Anreicherung eines durch Metabolisierung der Wirksubstanz erzeugten Photosensibilisators in den proliferierenden Zellen, verglichen mit normalen Zellen. Vorzugsweise ist die Wirksubstanz 5-Aminolävulinsäure und der Photosensibilisator Protoporphyrin IX. Die Anreicherung des Photosensibilisators kann durch photodiagnostische Verfahren bestimmt werden, z.B. durch Bestrahlen mit Licht mit 405 nm Wellenlänge und Messen der durch den Photosensibilisator erzeugten Fluoreszenzstrahlung. Die erfindungsgemäßen Nanoemulsionen sind insbesondere zur Verwendung in der Tumordiagnostik geeignet.

Ein weiterer Gegenstand der Erfindung ist die Verwendung der erfindungsgemäßen Nanoemulsion zur Herstellung eines Medikaments für die photodynamische Therapie.

Schließlich betrifft die Erfindung einen Kit, welcher eine erfindungsgemäße, zur topischen Applikation geeignete Nanoemulsion sowie eines oder mehrere Hilfsmittel enthält. Solche Hilfsmittel sind beispielsweise ein Abdeckmaterial, wie etwa eine Plastikfolie, das nach dem Aufbringen der Nanoemulsion auf die zu behandelnde Stelle aufgebracht wird, um eine vorzeitige Aktivierung durch Licht zu verhindern, Mittel zur Befestigung des Abdeckmaterials oder auch Mittel zum Auftragen der Nanoemulsion auf die zu behandelnde Stelle.

Die nachfolgenden Beispiele sollen die Erfindung weiterhin erläutern.

### Beispiele

### 1. Herstellung einer 10% 5-Aminolävulinsäure-Lotion

Es wurde ein Nanokolloidträgersystem gemäß dem Verfahren von US-Patent-Nr. 5,152,923 aus Eilecithin (83% Phosphatidylcholin), Miglyol 812 (Triglycerid) und Polysorbatum 80 in einem Phosphatpuffer hergestellt. Die Analysedaten des Trägersystems waren wie in Tabelle 1 angegeben.

**Tabelle 1**

| Wässrige Phase | Phosphatpuffer 20 mM, pH 6,0 |
|---|---|
| optische Eigenschaften | gelbe, stark schillernde Flüssigkeit |
| pH bei Raumtemperatur | 6,0 |
| Viskosität (20°C) | 1,6 mPas |
| Größe der Nanopartikel | ≤ 10-200 nm |
| mittlerer Durchmesser | 19,4 nm |
| Eilecithin-Gehalt | 17,5 mg/ml |
| Polysorbatum 80 Gehalt | ≤ 3% (w/w) |
| Miglyol 812 Gehalt | 34-38 mg/ml |
| Aerobe mesophile Keime in 50 ml | < 1 CFU/ml |

Die zur Herstellung einer 5-ALA-Nanokolloid-Lotion verwendeten Komponenten und deren relative Anteile sind in Tabelle 2 angegeben.

**Tabelle 2**

| Bezeichung | Menge (Gew.-%) |
|---|---|
| **Phase 1** | |
| Cetylalkohol | 5,00 % |
| Stearylalkohol | 1,00 % |
| Glycerinmonostearat | 2,00 % |
| Vaselinfett SNOWWHITE | 2,00 % |
| Paraffin pharm.perliquidum | 2,00 % |
| Isopropylmyristat kosm. | 4,00 % |
| Cremophor A 25 | 1,00 % |
| Cremophor S9 | 1,50 % |
| Cremophor EL 00647 | 1,88 % |
| **Phase 2** | |
| Wasser | 48,87 % |
| Sorbitol 70 % | 0.25 % |
| Phenoxyethanol | 0,50 |
| **Phase 3** | |
| Nanokolloid (Tabelle 1) | 20,00 % |
| 5-Aminolävulinsäure-hydrochlorid | 10,00 % |

Sämtliche Verfahrensschritte wurden unter Ausschluß von Licht und Luftsauerstoff durchgeführt. Phase 1 wurde hergestellt durch Zusammenschmelzen der Komponenten gemäß Tabelle 2 in den angegebenen Mengenverhältnissen bei 80°C und anschließendem Mischen.

Phase 2 wurde in einem separaten Gefäß vorbereitet. Hierzu wurde das Wasser vorgelegt und die übrigen Komponenten gemäß Tabelle 2 unter Rühren zugegeben. Nach ausreichendem Mischen wurde Phase 2 auf 80°C erwärmt und unter Vakuum zu Phase 1 zugemischt.

Nach Abkühlen auf 75°C wurde das Gemisch 2 min lang in einem Homogenisator homogenisiert. Das entstandene Gemisch wurde auf 60°C abgekühlt und erneut 2 min lang homogenisiert.

Phase 3 wurde in einem separaten Gefäß unter Vakuum und Lichtausschluß hergestellt. Hierzu wurde das Nanokolloid-Trägersystem wie vorstehend beschrieben vorgelegt und das 5-Aminolävulinsäurehydrochlorid bei 25 bis 30°C darin gelöst. Anschließend wurde Phase 3 bei 40°C unter Vakuum zum Gemisch der Phasen 1 und 2 zugegeben. Die Zusammensetzung wurde danach mit Schutzgas begast und 2 bis 3 min lang in einem Homogenisator homogenisiert. Anschließend wurde unter Rühren auf Raumtemperatur abkühlen gelassen.

Zur Bestimmung der Langzeitstabilität des Wirkstoffs wurde ein Teil der Lotion einem Lagerungstest unterzogen. Der 5-ALA-Gehalt betrug nach einjähriger Lagerung bei 5°C 97,92% des ursprünglichen Gehalts, bei Raumtemperatur wurde im selben Zeitraum ein Wert von 96,50% erhalten.

### 2. Herstellung eines 10% 5-Aminolävulinsäure-Gels

Es wurde ein Nanokolloidträgersystem gemäß dem Verfahren von US-Patent Nr. 5,152,923 aus Eilecithin und Miglyol 812 (Triglycerid) in einem K/Na-Phosphatpuffer hergestellt. Die Analysedaten waren wie in Tabelle 3 angegeben.

**Tabelle 3**

| wässrige Phase | Phosphatpuffer 20 mM, pH 6,0 |
|---|---|
| optische Eigenschaften | milchige Flüssigkeit |
| pH bei Raumtemperatur | 6,0 |
| Viskosität bei 20 °C | 1,5 mPas |
| Größe der Nanopartikel | ≤ 10-200 nm |
| mittlerer Durchmesser | 110,6 nm |
| Standardabweichung | 32,1 % |
| Gesamtlipid-Gehalt | 105,4 mg/g |
| Lecithin-Gehalt | 27,0 mg/g |
| Miglyol 812-Gehalt | 78,4 mg/g |
| aerobe mesophile Keime in 100 ml | < 1 CFU/ml |

Die zur Herstellung eines 5-Aminolävulinsäure-Nanokolloid-Gels verwendeten Komponenten und deren relative Anteile sind in Tabelle 4 angegeben.

**Tabelle 4**

| **Bezeichnung** | **Menge (Gew.-%)** |
|---|---|
| **Phase 1** | |
| Wasser | 38,30 % |
| Keltrol | 1 ,70 % |
| **Phase 2** | |
| Nanokolloid (Tabelle 3) | 50,00 % |
| Aminolävulinsäure-hydrochlorid | 10,00 % |

Zur Herstellung von Phase 1 wurde das Wasser vorgelegt, auf 60 bis 70°C erwärmt und anschließend Keltrol unter Vakuum darin dispergiert. Das Gemisch wurde 4 min bei Stufe 1 homogenisiert, danach unter Rühren auf Stufe 1 auf 30°C abkühlen gelassen.

Zur Herstellung von Phase 2 wurde das Nanoträgersystem in einem verschlossenen Gefäß unter Vakuum bei Raumtemperatur vorgelegt, und das 5-Aminolävulinsäurehydrochlorid wurde unter Rühren in 2 bis 3 h vollständig gelöst.

Danach wurde Phase 2 unter Vakuum zu Phase 1 gemischt und anschließend mit Stickstoff begast. Die entstandene Zusammensetzung wurde bei einer Temperatur von maximal 30°C 2 h unter Rühren homogen gemischt.

Zur Bestimmung der Langzeitstabilität des Wirkstoffs wurde ein Teil des Gels einem Lagerungstest unterzogen. Der 5-ALA-Gehalt betrug nach einjähriger Lagerung bei 5°C 99,17% des ursprünglichen Gehalts, bei Raumtemperatur wurde im gleichen Zeitraum ein Wert von 98,94% erhalten.

### 3. Photodymamische Therapie unter Verwendung der Nanokolloid-Lotion von Beispiel 1

Die Wirkung der erfindungsgemäßen Nanolotion wurde in einer klinischen Studie an 55 Basalzellkarzinomen in einem Patientenkollektiv von 19 Personen untersucht.

Vor Auftragen der Nanokolloidlotion wurde die gesamte zu behandelnde Hautfläche mit einer alkoholischen Lösung gereinigt. Es wurden jeweils 0,15 g Nanokolloidlösung pro cm² der zu behandelnden Hautfläche aufgebracht, was zu einem dünnen, sichtbaren Lotionsfilm führte. Nach dem Auftragen wurde die gesamte Fläche mit einem lichtundurchsichtigen Abdeckmaterial abgedeckt, um ein Verschmieren der Lotion und durch Raumlicht hervorgerufene unerwünschte photodynamische Reaktionen zu verhindern. Nach einer Einwirkzeit von 6 h wurde die Abdeckung entfernt, und die Gegenwart von Protoporphyrin IX sowie das Ausmaß des Tumors wurden bewertet anhand der charakteristisch roten Fluoreszenz von Porphyrinen bei Bestrahlung mit ultraviolettem Licht.

Die Bestrahlung wurde durchgeführt mit unfiltriertem Licht aus einer 250 W Halogenlampe mit einer Spektralverteilung über den gesamten sichtbaren Bereich bei einem Maximum von etwa 800 nm. Alle Läsionen wurden in einem Abstand von 10 cm bestrahlt, was die Bestrahlung von Bereichen mit einem Durchmesser bis zu 10 cm erlaubte. Die Bestrahlungsdauer betrug 20 min bei einer Intensität von 200 mW/cm², entsprechend einer Gesamtlichtdosis von 240 J/cm².

Das Patientenkollektiv umfaßte 19 Personen, die ein oder mehrere oberflächliche Basalzellkarzinome ohne Metastasen hatten. Insgesamt wurden 55 Basalzellkarzinome mittels photodynamischer Therapie behandelt. Keiner der in diese Studie aufgenommenen Patienten war zuvor mit entweder einer konventionellen Methode oder photodynamischen Therapie behandelt worden. Das Alter der Patienten betrug 32 bis 93 Jahre, mit einem Durchschnittsalter von 65 Jahren. 14 (73,7%) Patienten waren männlich und 5 (25,3%) weiblich. Mit einer Ausnahme waren die in dieser Studie behandelten Hauttumoren oberflächliche Läsionen. Der mittlere Durchmesser der Läsionen betrug 13,2 mm, mit einer Größenvariation zwischen 4,0 und 45 mm. Die Zahl der behandelten Tumoren in unterschiedlichen Körperbereichen waren 11 (20%) im Kopf und Nackenbereich, 37 (67%) im Rumpfbereich, 3 (5%) auf den oberen Gliedmaßen und 4 (7%) auf den unteren Gliedmaßen. Vor Aufnahme der Behandlung wurden routinemäßig Biopsien entnommen um die Diagnose zu bestätigen. Der Therapieerfolg wurde bewertet durch visuelle Inspektion und Palpation und bei 26 (47%) der Tumoren auch durch histopathologische Untersuchungen. Die in Abwesenheit eines klinisch feststellbaren Tumors an der Behandlungsstelle bei der Nachuntersuchung wurde als Tumorvollresponse definiert. Eine merkliche Verringerung der Tumorgröße wurde als Tumorteilresponse definiert.

Die in dieser Studie erreichten Ansprechraten sind in Tabelle 5 zusammengefaßt. Es wurde festgestellt, daß 47 (85%) der 55 in 19 Patienten behandelten Basalzellkarzinome nach einer einzigen Behandlung vollständig zurückgingen, wie durch Nachuntersuchung nach mindestens 6 Monaten nach der Behandlung klinisch festgestellt. Für die restlichen 8 (15%) Basalkarzinome wurde ein Teilrückgang, d.h. eine merkliche Verringerung der Tumorgröße, festgestellt.

Tabelle 6 zeigt die Resultate der photodynamischen Therapie in Abhängigkeit von der Lokalisierung der Basalzellkarzinome. Die besten Resultate wurden für die sieben Läsionen auf den Gliedmaßen erhalten, die vollständig zurückgingen. Von 37 Rumpfläsionen gingen 32 Tumoren vollständig zurück und von 11 Tumoren im Kopf- und Nackenbereich gingen 8 (76%) vollständig zurück.

**Tabelle 5**

| | visuelle Beurteilung | Biopsie |
|---|---|---|
| Zahl der Patienten | 19 | 13 |
| Basalzellkarzinome | 55 | 26 |
| vollständig. Rückgang | 47 (85%) | 21 (81%) |
| Teilrückgang | 8 (15%) | 5 (19%) |
| keine Reaktion | - | - |

**Tabelle 6**

| | Kopf und Nacken | Rumpf | Gliedmaßen |
|---|---|---|---|
| Zahl der Patienten | 6 | 12 | 4 |
| Basalzellkarzinome | 11 | 37 | 7 |
| vollständ. Rückgang | 8 (73%) | 32 (86%) | 7 (100%) |
| Teilrückgang | 3 (27%) | 5 (14%) | - |
| keine Reaktion | - | - | - |

### 4. Photodynamische Therapie unter Verwendung des Nanokolloid-Gels gemäß Beispiel 2

Die Wirkung des erfindungsgemäßen Nanoemulsion in Form eines Gels wurde in einer klinischen Studie zur photodynamischen Therapie von Kondylomata acuminata und intraepithelialer Neoplasie der Vulva (VIN) an 47 Läsionen eine Patientenkollektivs von 16 Personen mit einem Alter von 18 bis 45 Jahren (Durchschnitssalter 32,7 Jahre) untersucht.

Das Auftragen des Gels erfolgte wie in Beispiel 3 für die Lotion beschrieben, mit der Ausnahme, daß die Inkubationszeit zur Diffusion der 5-Aminolävulinsäure lediglich 90 min betrug. Bestrahlung erfolgte unter Verwendung eines Argon-Farbstofflasers (Coherent Innova, Modell 310, Palo Alto, CA) mit monochromatischem Licht (630 nm) und mit Lichtdosen zwischen 50 J/cm² und 125 J/cm².

Nach einer einzigen Behandlung zeigten während einer Nachuntersuchungszeit zwischen 1 bis 12 Monaten neun der sechzehn Patienten einen vollständigen Rückgang und die anderen sieben einen Teilrückgang. Die Behandlung wurde vom Großteil des Patientenkollektivs gut vertragen. Den Patienten stand offen, die Bestrahlung zu unterbrechen, wenn der Schmerz übermäßig wurde. Die Anzahl der Unterbrechungen bis zum Erreichen der kompletten Strahlungsdauer wurde festgehalten. Lediglich drei der Patienten hatten die Bestrahlung öfter als fünfmal unterbrochen, fünf Patienten unterbrachen die Bestrahlung ein oder zweimal und die restlichen benötigten keine Unterbrechung.

## Patentansprüche

1. Zusammensetzung,
**dadurch gekennzeichnet,**
**daß** sie eine Nanoemulsion umfassend eine Wirksubstanz ausgewählt aus 5-Aminolävulinsäure, einem Salz, einem Komplex oder einer Additionsverbindung davon, oder/und zu Protoporphyrin IX umsetzbaren Vorstufen oder/und Metaboliten davon, und einen Träger in einer wässrigen Phase enthält, wobei der Träger aus einem oder mehreren Lipiden und aus einem oder mehreren Emulgatoren umfassend Sojalecithin gebildet ist.

2. Zusammensetzung nach Anspruch 1,
**dadurch gekennzeichnet,**
**daß** die mittlere Größe der emulgierten Teilchen 10 bis 200 nm beträgt.

3. Zusammensetzung nach einem der vorhergehenden Ansprüche,
**dadurch gekennzeichnet,**
**daß** die Wirksubstanz in einem Anteil von 1 bis 25 Gew.-%, insbesondere von 5 bis 15 Gew.-%, bezogen auf das Gesamtgewicht der Zusammensetzung vorhanden ist.

4. Zusammensetzung nach einem der vorhergehenden Ansprüche,
**dadurch gekennzeichnet,**
**daß** sie weiterhin Hilfs- oder/und Zusatzstoffe umfaßt, die in der Kosmetik oder Pharmazie üblich sind.

5. Zusammensetzung nach Anspruch 4,
**dadurch gekennzeichnet,**
**daß** sie in Form eines Gels vorliegt und, bezogen auf das Gesamtgewicht der Zusammensetzung, 5 bis 15% Wirksubstanz, 45 bis 55% Träger, 1 bis 5% Hilfsstoffe und den Rest Wasser umfaßt.

6. Zusammensetzung nach Anspruch 4,
**dadurch gekennzeichnet,**
**daß** sie in Form einer Lotion vorliegt und, bezogen auf das Gesamtgewicht der Zusammensetzung, 5 bis 15% Wirksubstanz, 15 bis 25% Träger, 15 bis 25% Hilfsstoffe und den Rest Wasser umfaßt.

7. Zusammensetzung nach einem der vorhergehenden Ansprüche,
**dadurch gekennzeichnet,**
**daß** der Gehalt an Wirksubstanz nach einjähriger Lagerung bei Raumtemperatur von nicht mehr als 5% verringert ist.

8. Zusammensetzung nach einem der vorhergehenden Ansprüche,
**dadurch gekennzeichnet,**
**daß** sie in Form eine pharmazeutischen Präparats ist.

9. Zusammensetzung nach Anspruch 8,
**dadurch gekennzeichnet,**
**daß** sie topisch applizierbar ist.

10. Verfahren zur Herstellung einer Zusammensetzung nach einem der vorhergehenden Ansprüche,
**dadurch gekennzeichnet,**
**daß** man ein Gemisch, umfassend einen Träger und eine wässrige Phase herstellt und in eine Nanoemulsion überführt, wobei vor oder/und nach der Überführung in die Nanoemulsion die Wirksubstanz zugegeben wird, und man danach gegebenenfalls Hilfs- oder/und Zusatzstoffe beimischt.

11. Verfahren nach Anspruch 10,
**dadurch gekennzeichnet,**
**daß** man unter Sauerstoff- oder/und Lichtausschluß arbeitet.

12. Verfahren nach Anspruch 10 oder 11,
**dadurch gekennzeichnet,**
**daß** man bei einer Temperatur von 5 bis 45°C arbeitet.

13. Verwendung einer Zusammensetzung nach einem der Ansprüche 1 - 9 zur Herstellung eines Mittels für die photodynamische Therapie.

14. Verwendung nach Anspruch 13,
**dadurch gekennzeichnet,**
**daß** die Zusammensetzung topisch appliziert wird.

15. Verwendung nach Anspruch 13 oder 14 zur Herstellung eines Mittels für die Therapie von mit Zellproliferation assoziierten Erkrankungen.

16. Verwendung nach Anspruch 15 zur Herstellung eines Mittels für die Therapie von Tumorerkrankungen.

17. Verwendung nach Anspruch 16,
**dadurch gekennzeichnet,**
**daß** die Erkrankung ein Basalzellkarzinom, ein Plattenepithelkarzinom, Morbus Bowen, aktinische Keratose, Condylomata acuminata (CIN), intraepitheliale Neoplasie der Vulva (VIN) oder eine knotenartige oder subkutane Krebserkrankung ist.

18. Verwendung nach Anspruch 15,
**dadurch gekennzeichnet,**
**daß** die Erkrankung Psoriasis ist.

19. Verwendung nach Anspruch 13,
**dadurch gekennzeichnet,**
**daß** man die Zusammensetzung nach einem der Ansprüche 1 - 9 einem erkrankten Lebewesen in einer wirksamen Menge verabreicht, für einen Zeitraum inkubiert, der geeignet ist um für eine ausreichende Menge der Wirksubstanz in dem zu behandelnden Gewebe zu sorgen und das Gewebe mit Licht bestrahlt.

20. Verwendung einer Zusammensetzung nach einem der Ansprüche 1 - 9 zur Herstellung eines Mittels zum Nachweis von proliferierenden Zellen.

21. Verwendung nach Anspruch 20 zur Herstellung eines Mittels zur Diagnose von Tumorerkrankungen.

22. Kit, umfassend eine topisch applizierbare Zusammensetzung nach Anspruch 9 und mindestens eine Komponente, ausgewählt aus
(a) einem im wesentlichen lichtundurchlässigen blattartigen Material,
(b) Mittel zur Befestigung des blattartigen Materials auf einem Applikationsort, und
(c) Mittel zum Auftragen der Zusammensetzung auf einem Applikationsort.

## Claims

1. Composition,
**characterized in that**
it contains a nanoemulsion which comprises an active substance selected from 5-aminolevulinic acid, or a salt, a complex or an addition compound thereof, and/or precursors and/or metabolites thereof which can be converted into protoporphyrin IX, and a carrier in an aqueous phase, with the carrier being formed from one or more lipids and from one or more emulsifiers including soybean lecithin.

2. Composition according to Claim 1,
**characterized in that**
the average size of the emulsified particles is from 10 to 200 nm.

3. Composition according to one of the preceding claims,
**characterized in that**
the active substance is present in a proportion of from 1 to 25% by weight, in particular of from 5 to 15% by weight, based on the total weight of the composition.

4. Composition according to one of the preceding claims,
**characterized in that**
it additionally comprises adjuvants and/or additives which are customary in cosmetics or pharmacy.

5. Composition according to Claim 4,
**characterized in that**
it is present in the form of a gel and, based on the total weight of the composition, comprises from 5 to 15% of active substance, from 45 to 55% of carrier and from 1 to 5% of adjuvants, with the remainder being water.

6. Composition according to Claim 4,
**characterized in that**
it is present in the form of a lotion and, based on the total weight of the composition, comprises from 5 to 15% of active substance, from 15 to 25% of carrier and from 15 to 25% of adjuvants, with the remainder being water.

7. Composition according to one of the preceding claims,
**characterized in that**
the content of active substance is reduced by not more than 5% after one year of storage at room temperature.

8. Composition according to one of the preceding claims,
**characterized in that**
it is in the form of a pharmaceutical preparation.

9. Composition according to Claim 8,
**characterized in that**
it can be applied topically.

10. Process for preparing a composition according to one of the preceding claims,
**characterized in that**
a mixture comprising a carrier and an aqueous phase is prepared and converted into a nanoemulsion, with the active substance being added before and/or after the conversion into the nanoemulsion, and, after that, adjuvants and/or additives are admixed where appropriate.

11. Process according to Claim 10,
**characterized in that**
it is carried out while excluding oxygen and/or light.

12. Process according to Claim 10 or 11,
**characterized in that**
it is carried out at a temperature of from 5 to 45°C.

13. Use of a composition according to one of Claims 1-9 for preparing an agent for photodynamic therapy.

14. Use according to Claim 13,
**characterized in that**
the composition is applied topically.

15. Use according to Claim 13 or 14 for preparing an agent for the therapy of diseases which are associated with cell proliferation.

16. Use according to Claim 15 for preparing an agent for the therapy of tumour diseases.

17. Use according to Claim 16,
**characterized in that**
the disease is a basal cell carcinoma, a squamous cell carcinoma, Bowen's disease, solar keratosis, condylomata acuminata (CIN), intraepithelial neoplasia of the vulva (VIN), or a nodose or subcutaneous cancer disease.

18. Use according to Claim 15,
**characterized in that**
the disease is psoriasis.

19. Use according to Claim 13,
**characterized in that**
the composition according to one of Claims 1-9 is administered in an effective quantity to a diseased organism, incubation is performed for a period which is suitable for ensuring that an adequate quantity of the active substance is present in the tissue being treated, and the tissue is irradiated with light.

20. Use of a composition according to one of Claims 1-9 for preparing a means for detecting proliferating cells.

21. Use according to Claim 20 for preparing a means for diagnosing tumour diseases.

22. Kit which comprises a topically applicable composition according to Claim 9 and at least one component selected from
(a) an essentially light-impermeable sheet-like material,
(b) means for attaching the sheet-like material to a site of application, and
(c) means for applying the composition to a site of application.

## Revendications

1. Composition, **caractérisée en ce qu'**elle contient une nanoémulsion comprenant une substance active choisie parmi l'acide 5-aminolévulinique, un de ses sels, de ses complexes ou de ses composés d'addition, et/ou les précurseurs que l'on peut transformer en protoporphyrine IX et/ou leurs métabolites, et un support dans une phase aqueuse, le support étant formé à partir d'un ou de plusieurs lipides et d'une lécithine de soja comprenant un ou plusieurs émulsifiants.

2. Composition selon la revendication 1, **caractérisée en ce que** la granulométrie moyenne des particules émulsionnées va de 10 à 200 nm.

3. Composition selon une quelconque des revendications précédentes, **caractérisée en ce que** la substance active est présente à raison de 1 à 25% en poids, en particulier à raison de 5 à 15% en poids, par rapport au poids total de la composition.

4. Composition selon une quelconque des revendications précédentes, **caractérisée en ce qu'**elle contient en outre des adjuvants ou/et des additifs qui sont couramment employés dans les produits cosmétiques ou pharmaceutiques.

5. Composition selon la revendication 4, **caractérisée en ce qu'**elle se présente sous forme d'un gel et qu'elle comprend, rapporté au poids total de la composition, la substance active à raison de 5 à 15%, le support à raison de 45 à 55%, les adjuvants à raison de 1 à 5%, et le complément étant de l'eau.

6. Composition selon la revendication 4, **caractérisée en ce qu'**elle se présente sous forme d'une lotion et qu'elle comprend, rapporté au poids total de la composition, la substance active à raison de 5 à 15%, le support à raison de 15 à 25%, les adjuvants à raison de 15 à 25%, et le complément étant de l'eau.

7. Composition selon une quelconque des revendications précédentes, **caractérisée en ce que**, à l'issue d'un stockage d'une durée d'un an à température ambiante, la diminution de la teneur en substance active reste inférieure ou égale à 5%.

8. Composition selon une quelconque des revendications précédentes, **caractérisée en ce qu'**elle se présente sous forme d'une préparation pharmaceutique.

9. Composition selon la revendication 8, **caractérisée en ce qu'**elle convient à une application topique.

10. Procédé de préparation d'une composition selon une quelconque des revendications précédentes, **caractérisé en ce que** l'on prépare un mélange comprenant un support et une phase aqueuse, qu'on le transforme en une nanoémulsion, la substance active étant ajoutée avant et/ou après la transformation en nanoémulsion, **en ce que** l'on ajoute par mélange éventuellement ensuite des adjuvants et/ou des additifs.

11. Procédé selon la revendication 10, **caractérisé en ce que** l'on procède en l'absence d'oxygène et/ou de lumière.

12. Procédé selon la revendication 10 ou 11, **caractérisé en ce que** l'on procède à une température de 5 à 45°C.

13. Utilisation d'une composition selon une quelconque des revendications 1 à 9, pour la préparation d'un moyen pour la thérapie photodynamique.

14. Utilisation selon la revendication 13, **caractérisée en ce que** la composition est appliquée de façon topique.

15. Utilisation selon la revendication 13 ou 14, pour la préparation d'un moyen pour le traitement de maladies associées à une prolifération cellulaire.

16. Utilisation selon la revendication 15, pour la préparation d'un moyen pour le traitement de maladies tumorales.

17. Utilisation selon la revendication 16, **caractérisée en ce que** la maladie est un carcinome de cellules basales, un carcinome de l'épithélium pavimenteux, la maladie de Bowen, la kératose actinique, le condylome acuminé (CIN), une néoplasie intraépithéliale vulvaire (VIN) ou une maladie cancéreuse sous cutanée ou nodulaire.

18. Utilisation selon la revendication 15, **caractérisée en ce que** la maladie est le psoriasis.

19. Utilisation selon la revendication 13, **caractérisée en ce que** l'on procède à une administration, en quantité efficace, de la composition selon une quelconque des revendications 1 à 9, à un être vivant malade, avec une incubation pendant une durée permettant l'installation de la substance active, en quantité suffisante, dans le tissu à traiter, et **en ce que** l'on expose ce tissu à la lumière.

20. Utilisation d'une composition selon une quelconque des revendications 1 à 9, pour la préparation d'un moyen pour la mise en évidence des cellules proliférantes.

21. Utilisation selon la revendication 20 pour la préparation d'un moyen pour le diagnostic de maladies tumorales.

22. Kit comprenant une composition qui convient à l'application topique selon la revendication 9 ainsi qu'au moins un composant choisi parmi :
(a) un matériau foliacé essentiellement opaque,
(b) un moyen pour la fixation, sur un emplacement d'application, du matériau foliacé,
(c) un moyen servant à l'application de la composition à l'emplacement d'application.
